# EUROPEAN PATENT APPLICATION

(11) **EP 4 660 635 A1**
(43) Date of publication of application: **10.12.2025**
(21) Application number: 24749909.8
(22) Date of filing: 12.01.2024
(51) Int. Cl.: G01N 35/10, G01N 1/10

(54) **NOZZLE ASSEMBLY AND INSPECTION DEVICE**

(30) Priority: 30.01.2023 JP 2023012145
(71) Applicant: FUJIFILM Corporation, Tokyo 106-8620 (JP)
(72) Inventor: HIBE, Daisuke, Ashigarakami-gun, Kanagawa 258-8538 (JP)
(74) Representative: Dehns Germany Partnerschaft mbB
(86) International application number: PCT/JP2024/000681
(87) International publication number: WO 2024/161954

(57) **Abstract**

In a nozzle assembly and an examination apparatus, the nozzle assembly is a nozzle assembly in which two nozzles with circular cross sections are integrated in a state of being brought into contact with each other, in which a recess portion that is generated in a part of an outer periphery of the two nozzles in contact with each other and that has a valley at a contact portion where the nozzles are brought into contact with each other is flattened by being filled with a filling member, and a part of the outer periphery other than the recess portion, the part including both end parts of the outer periphery in a maximum diameter direction, is not covered by the filling member.

## Description

### BACKGROUND OF THE INVENTION

### 1. Field of the Invention

The present disclosure relates to a nozzle assembly and an examination apparatus.

### 2. Description of the Related Art

An examination apparatus (also referred to as an analysis apparatus) that quantitatively or qualitatively detects a target substance in a specimen is known. Many of such examination apparatuses use an immunoassay principle, and examples thereof include a chemiluminescent enzyme immunoassay apparatus and a fluorescent immunoassay apparatus.

These examination apparatuses execute a detection process of detecting a target substance in a specimen by detecting luminescence or fluorescence based on a label such as an enzyme label or a fluorescent label attached to the target substance in the specimen by using an immunoreaction. In addition, before such a detection process of the target substance, a pre-process such as attachment of a label to the target substance in the specimen is executed on the specimen. In some cases, the examination apparatus is configured to automate the pre-process and the detection process, so that in a case in which a specimen collection container containing a collected specimen is loaded, the pre-process and the detection process are automatically executed and detection results are output.

An examination using an immunoreaction includes a bound/free (BF) separation step for removing an unnecessary component after binding a capture antibody immobilized in a solid phase to an antigen as a detection target substance and for removing a labeled antibody that is not bound to the detection target substance after binding the detection target substance captured by the capture antibody to a labeled antibody. In the examination apparatus, it is desired to perform the BF separation quickly and stably in order to improve the throughput of the examination and the examination accuracy.

JP2015-230181A proposes a collective nozzle (corresponding to a nozzle assembly) in which a suction nozzle and a jetting nozzle are bundled and integrated to efficiently perform the BF separation.

JP2016-085093A proposes an examination apparatus that performs the BF separation using magnetic particles. During the BF separation, a nozzle is inserted into a reaction solution in a reaction container, and the reaction solution in the reaction container is suctioned by the nozzle. In this case, in order to prevent the magnetic particles that have formed immune complexes contained in the reaction solution from being suctioned, the reaction solution is suctioned in a state where the magnetic particles are temporarily attracted to an inner wall surface of the reaction container by a magnet disposed outside the reaction container, that is, in a magnetically collected state. As a result, only magnetic particles that are bound to an intended substance to form immune complexes are left in the reaction container, and other unreacted specimen-derived components and the like are removed through the suctioned solution. Thereafter, a washing solution is discharged and suctioned through the nozzle to and from the reaction container, and the magnetic particles in the reaction container are washed.

### SUMMARY OF THE INVENTION

In a case in which the collective nozzle as in JP2015-230181A is used, the BF separation step can be efficiently performed even in the BF separation using the magnetic particles disclosed in JP2016-085093A. On the other hand, there are the following problems.

In the collective nozzle in which the suction nozzle and the jetting nozzle are integrated, as disclosed in JP2015-230181A, the suction nozzle and the jetting nozzle are fixed by a fixing portion, and upper parts of both nozzles protruding from a lower end of the fixing portion are covered with a cover. Since the two nozzles are integrated and covered with a cover, a cross-sectional ratio of the nozzle to a cross section of the reaction container is larger than a cross section of single suction nozzle, and in a case in which the nozzle is inserted into the reaction container, a distance to the magnetic particles magnetically collected on the inner wall surface is small, making it easier for the magnetic particles to be erroneously suctioned.

The present disclosure has been made in view of the above circumstances, and an object of the present disclosure is to provide a nozzle assembly and an examination apparatus capable of, in a case of performing BF separation using magnetic particles in an examination using an immunoreaction, efficiently performing the BF separation and suppressing erroneous suction of the magnetic particles.

A nozzle assembly of the present disclosure is a nozzle assembly in which two nozzles with circular cross sections are integrated in a state of being brought into contact with each other, in which a recess portion that is generated in a part of an outer periphery of the two nozzles in contact with each other and that has a valley at a contact portion where the nozzles are in contact with each other is flattened by being filled with a filling member, and a part of the outer periphery other than the recess portion, the part including both end parts of the outer periphery in a maximum diameter direction, is not covered by the filling member.

It is preferable that the filling member is provided only in the recess portion.

It is preferable that the nozzle assembly has an oval cross-sectional shape in a state where the recess portion is flattened.

It is preferable that the two nozzles are made of metal.

It is preferable that the filling member is made of metal.

It is preferable that the metal constituting the filling member is silver braze or stainless steel.

It is preferable that tip positions of the two nozzles are aligned.

It is preferable that the recess portion is filled with the filling member from a position separated from the tip positions by 3 mm or more.

It is preferable that water-repellent coating is applied to an entire outer periphery including the filling member.

It is preferable that the water-repellent coating is fluorine-based coating.

The nozzle assembly of the present disclosure may be used for suctioning and discharging a washing solution in a BF separation step in an examination using an immunoreaction.

An examination apparatus of the present disclosure is an examination apparatus that performs an examination using an immunoreaction and that performs an examination including a step of, using magnetic particles as a solid phase, binding a detection target substance in a specimen to a binding substance attached to the magnetic particles in a reaction container and then performing BF separation, the examination apparatus comprising: a reaction processing section that causes an immunoreaction in the reaction container; and a washing processing section that includes a nozzle assembly for suctioning and discharging a BF separation solution for performing the BF separation, in which, as the nozzle assembly, the nozzle assembly according to the present disclosure is provided.

It is preferable that the reaction container has an elliptical shape in a plan view, and that the washing processing section includes a magnet for magnetically collecting the magnetic particles on an inner wall surface of the reaction container in a minor axis direction.

According to the technology of the present disclosure, it is possible to provide a nozzle assembly and an examination apparatus capable of, in a case of performing BF separation using magnetic particles in an examination using an immunoreaction, efficiently performing the BF separation and suppressing erroneous suction of the magnetic particles.

### BRIEF DESCRIPTION OF THE DRAWINGS

Fig. 1 is a perspective view of a nozzle assembly.
Fig. 2 is a front view of the nozzle assembly.
Fig. 3 is a cross-sectional view of a nozzle portion of the nozzle assembly.
Fig. 4 is an explanatory diagram of a recess portion generated in a case in which two nozzles are brought into contact with each other.
Fig. 5 is a diagram showing a recess portion flattened by a filling member of a modification example.
Fig. 6 is a diagram showing a recess portion flattened by a filling member of a modification example.
Fig. 7 is a view showing a nozzle portion of a modification example.
Fig. 8 is a perspective view showing a nozzle assembly of a modification example.
Fig. 9 is a perspective view showing a nozzle assembly of a modification example.
Fig. 10 is a schematic diagram showing an overall configuration of an examination apparatus.
Fig. 11 is an explanatory view of a procedure of chemiluminescent enzyme immunoassay.
Fig. 12 is a diagram showing a state where the nozzle assembly is inserted into a reaction container.
Fig. 13 is a cross-sectional view of the nozzle portion and the reaction container in the state of Fig. 12.
Fig. 14 is a perspective view showing a state where two nozzles are brought into contact with each other.
Fig. 15 is a cross-sectional view of a nozzle portion of a nozzle assembly of a comparative example and the reaction container in a state where the nozzle portion is inserted into the reaction container.

### DESCRIPTION OF THE PREFERRED EMBODIMENTS

Hereinafter, an examination apparatus according to an embodiment of the present disclosure will be described with reference to the drawings. The constituent elements indicated by the same reference numerals in the drawings mean the same constituent elements. Unless otherwise specified in the specification, each constituent element is not limited to one, and each constituent element may be plural.

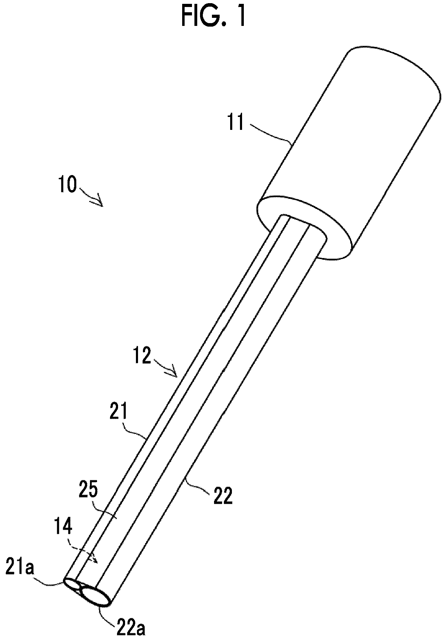

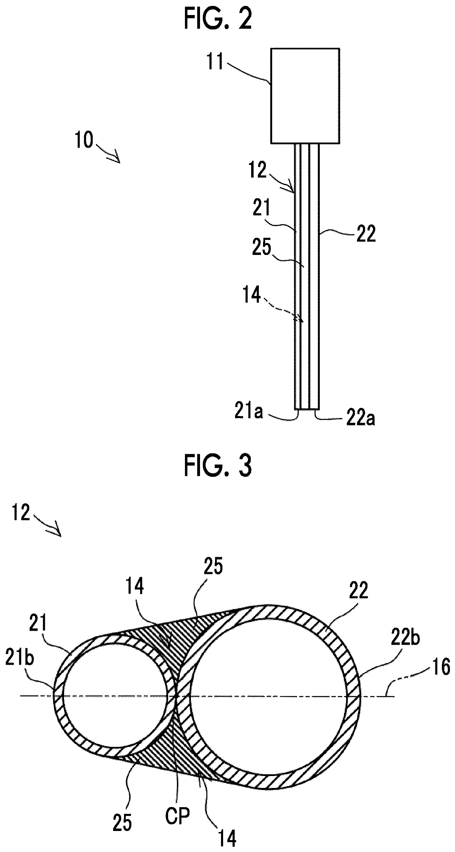

Fig. 1 is a perspective view of a nozzle assembly 10. Fig. 2 is a front view of the nozzle assembly 10. Fig. 3 is a cross-sectional view of a nozzle portion 12 of the nozzle assembly 10.

As shown in Fig. 1, the nozzle assembly 10 is a nozzle assembly in which two nozzles 21 and 22 with circular cross sections are integrated in a state of being brought into contact with each other. The nozzle assembly 10 of the present embodiment comprises a fixing portion 11 and a nozzle portion 12 that protrudes from the fixing portion 11. Hereinafter, one of the two nozzles 21 and 22 is referred to as a first nozzle 21, and the other is referred to as a second nozzle 22.

In this example, the first nozzle 21 and the second nozzle 22 are brought into contact with each other and integrated such that positions of their respective tips 21a and 22a are aligned. Note that the position of the tip 21a of the first nozzle 21 and the position of the tip 22a of the second nozzle 22 do not need to be completely aligned, and may be shifted from each other.

Base end sides of the first nozzle 21 and the second nozzle 22 are inserted into and supported by a tubular fixing portion 11, for example. Although not shown, the fixing portion 11 is provided with a pipe for supplying a liquid from the outside or for draining the liquid to the outside, and the base end sides of the first nozzle 21 and the second nozzle 22 are connected to the pipe.

In the nozzle assembly 10, a recess portion 14 that is generated on a part of an outer periphery of the two nozzles 21 and 22 in the contact state and that has a valley at a contact portion CP (see Fig. 3) where the nozzles 21 and 22 are brought into contact with each other is flattened by being filled with a filling member 25. On the other hand, in the nozzle assembly 10, a part of the outer periphery of the two nozzles 21 and 22 other than the recess portion 14, the part including both end parts of the outer periphery in the maximum diameter direction, is not covered by the filling member 25. The outer peripheries of the two nozzles 21 and 22 in the contact state are composed of an outer periphery 21b of the first nozzle 21 and an outer periphery 22b of the second nozzle 22, and have a figure eight shape in a cross section. The maximum diameter direction of the nozzle assembly 10 refers to a direction in which a straight line 16 connecting centers of the two nozzles 21 and 22 in contact with each other extends (see Fig. 3).

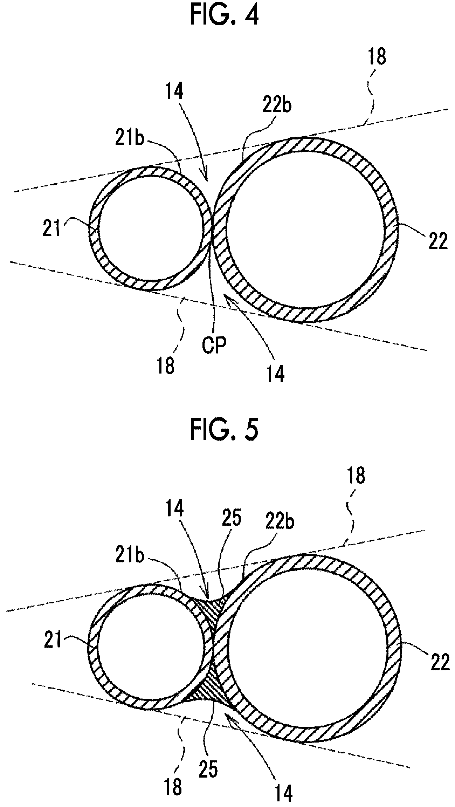

Fig. 4 shows a cross section of the nozzle portion 12 in a state where the two nozzles 21 and 22 are brought into contact with each other. As shown in Fig. 4, the recess portion 14 that is generated in a part of the outer periphery of the two nozzles 21 and 22 in the contact state refers to a portion surrounded by a common tangent 18 that contacts each of the first nozzle 21 and the second nozzle 22, and the outer periphery 21b of the first nozzle 21 and the outer periphery 22b of the second nozzle 22.

The nozzle assembly 10 of the present embodiment has an oval cross-sectional shape in a state where the recess portion 14 is flattened, as shown in Fig. 3. Here, the term "oval shape" is a general term for a flat shape relative to a circle, and includes an egg shape, an elliptical shape, a rounded rectangular shape, and shapes similar to these shapes.

It is preferable that the first nozzle 21 and the second nozzle 22 are made of metal. As the metal forming the first nozzle 21 and the second nozzle 22, stainless steel is suitably used.

It is also preferable that the filling member 25 is made of metal. As the metal used for the filling member 25, silver braze or stainless steel is preferable.

Since the nozzle assembly 10 of the present embodiment is a nozzle assembly in which two nozzles are integrated in a state of being brought in contact with each other, for example, in a case of BF separation in immunoassay, suction of a liquid and discharging of a BF separation solution (washing solution) can be efficiently performed. In a case in which a suction nozzle and a jetting nozzle are individually operated, it is necessary to perform an operation of inserting the suction nozzle into a reaction container, suctioning a liquid, taking out the suction nozzle, and then replacing the suction nozzle with the jetting nozzle. On the other hand, in a case in which the nozzle assembly 10 of the present embodiment is used, the two nozzles can be operated as a single body. Therefore, in a case in which one nozzle is used as the suction nozzle and the other nozzle is used as the jetting nozzle, an operation of replacing the suction nozzle and the jetting nozzle is not necessary.

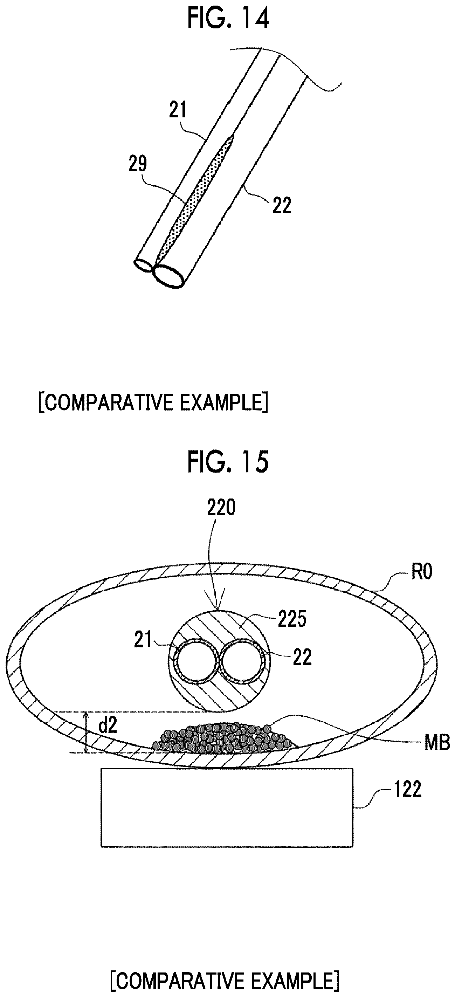

In a case in which the two nozzles 21 and 22 are brought into contact with each other and integrated, the recess portion 14 having a valley at the contact portion CP is formed as shown in Fig. 3. In a case in which the recess portion 14 is not filled with the filling member 25, a liquid 29 is drawn up due to a capillary action as shown in Fig. 14. In a case in which the nozzle assembly 10 is used in a BF separation step in an examination apparatus such as an immunoassay apparatus, in a case in which the liquid 29 is drawn up, dilution of a reagent or the like, carryover, or the like may occur, resulting in a deterioration in measurement accuracy. In the nozzle assembly 10 of the present embodiment, the recess portion 14 is filled with the filling member 25 and is flattened, so that it is possible to prevent a liquid from being drawn up due to the capillary action. Therefore, it is possible to suppress the deterioration in measurement accuracy in immunoassay or the like. In addition, in the nozzle assembly 10, a part of the outer periphery of the two nozzles 21 and 22, the part including both end parts of the outer periphery in the maximum diameter direction in a state where the two nozzles 21 and 22 are brought into contact with each other, is not covered by the filling member 25. In order to prevent the formation of the recess portion 14 that is generated in a case in which the two nozzles are brought into contact with each other, it is also possible to consider a configuration in which the two nozzles are held by a holding member that covers the entire outer periphery of the two nozzles. However, in a case in which the entire outer periphery is covered, the outer periphery of the nozzle assembly 10 is increased compared to a state where the two nozzles 21 and 22 are simply brought into contact with each other. On the other hand, in the present embodiment, at least a part including both end parts in the maximum diameter direction is not covered by the filling member 25, so that an increase in the outer periphery can be suppressed.

In the example shown in Fig. 3, the entire recess portion 14 is filled with the filling member 25 and is flattened. However, as shown in Fig. 5, an aspect in which a part of the recess portion 14 is filled with the filling member 25 may be adopted. The recess portion 14 shown in Fig. 5 need only be filled with the filling member 25 until the recess portion 14 is flattened to an extent that the liquid can be prevented from being drawn up due to the capillary action. For example, in Fig. 5, a steep V-shaped valley in a cross section before the recess portion 14 is filled with the filling member 25 is filled to an extent that the valley becomes a gentle U-shape.

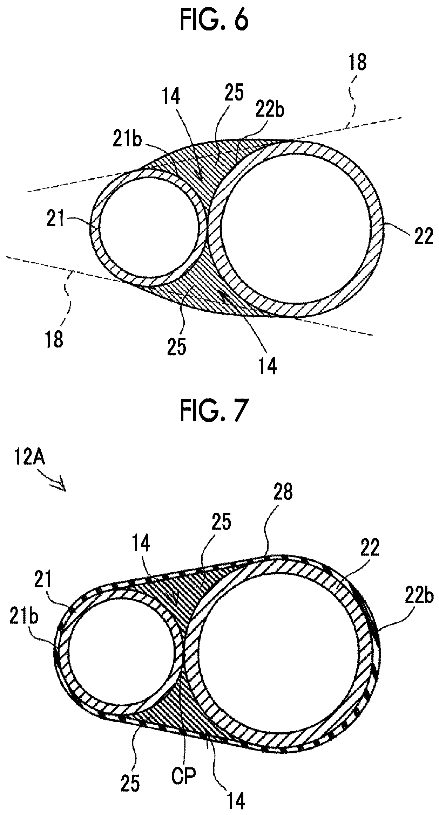

On the other hand, as shown in Fig. 6, the filling member 25 may have a portion that fills the recess portion 14 and that protrudes outward from the recess portion 14. Note that, from the viewpoint of suppressing the expansion of the outer periphery, it is preferable that the filling member 25 is provided only in the recess portion 14.

Fig. 7 shows a cross-sectional view of a nozzle portion 12A of a modification example. In the nozzle portion 12A, it is preferable that water-repellent coating is applied to the entire outer periphery including the first nozzle 21, the second nozzle 22, and the filling member 25 to provide a water-repellent coat 28.

It is preferable that the water-repellent coat 28 is formed of a fluorine-based coating agent.

In a case in which the entire outer periphery of the nozzle portion 12A is provided with the water-repellent coat 28, it is possible to suppress the adhesion of liquid droplets to an outer periphery surface of the nozzle portion 12A, and it is possible to reduce the liquid droplets remaining on the outer periphery surface in a case in which the nozzle portion 12A is pulled out of the liquid. In addition, in a case in which the filling member 25 is made of metal, the filling member 25 is covered with the water-repellent coat 28, so that the corrosion of the filling member 25 can be suppressed.

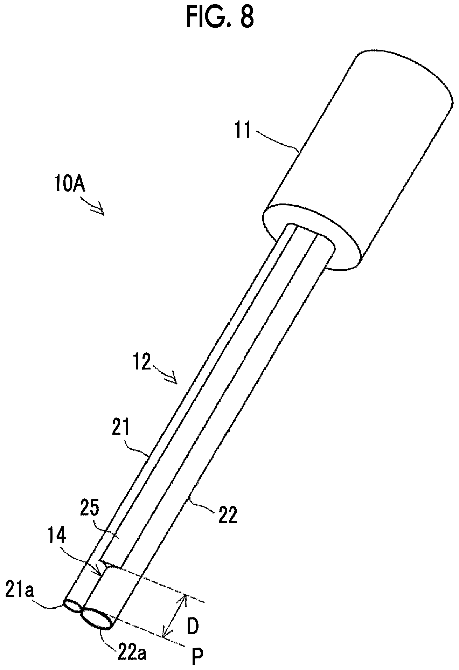

In the nozzle assembly 10 shown in Figs. 1 and 2, the filling member 25 fills the recess portion 14 over the entire region from the tip to the base end of the nozzle portion 12, but, as in a nozzle assembly 10A of a modification example shown in Fig. 8, the filling member 25 may fill the recess portion 14 on the base end side from a position separated by a distance D from a tip position P of the nozzle portion 12. In a case in which the position of the tip 21a of the first nozzle 21 and the position of the tip 22a of the second nozzle 22 are substantially aligned, the distance D is preferably 3 mm or more. The distance D is more preferably 15 mm or less and more preferably 10 mm or less.

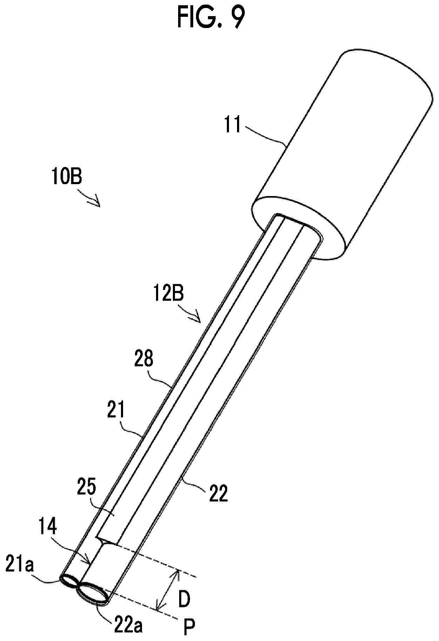

In particular, as in a nozzle assembly 10B of a modification example shown in Fig. 9, it is preferable that a nozzle portion 12B has a portion that is not filled with the filling member 25 in a region of 3 mm or more from a tip thereof, and that the entire region of the nozzle portion 12B is provided with the water-repellent coat 28.

The nozzle portions 12, 12A, and 12B of the nozzle assemblies 10, 10A, and 10B may be operated by bringing their tips into contact with the bottom of the reaction container, for example, by inserting the nozzle portions 12, 12A, and 12B into the reaction container and suctioning a liquid in the reaction container. In a case in which the water-repellent coat 28 of the tip portion that comes into contact with the reaction container is non-uniform, the water-repellent coat 28 is likely to be peeled off due to the contact.

As in the nozzle portion 12B of the nozzle assembly 10B shown in Fig. 9, by disposing the filling member 25 at a distance of 3 mm or more from the tip position P of the nozzle portion 12B, liquid pooling and the like are less likely to occur at the nozzle tip portion in a case of applying the water-repellent coating, making it easier to ensure surface uniformity and obtaining an effect of suppressing peeling upon contact. By suppressing the peeling of the water-repellent coat 28, in a case in which the filling member 25 is made of metal, corrosion of the metal can be suppressed. In this way, at the tip portion of the nozzle portion 12B, the filling member 25 is not provided, and the water-repellent coating is applied to the first nozzle 21 and the second nozzle 22 themselves, thereby making it possible to ensure the stability of the water-repellent coat 28. Since the filling member 25 fills the recess portion 14 other than the tip portion of the nozzle portion 12B, it is possible to prevent a liquid from being drawn up.

The nozzle assemblies 10, 10A, and 10B have a configuration in which two nozzles of the first nozzle 21 and the second nozzle 22 are provided, but may include three or more nozzles. In a case in which three or more nozzles are provided, the above-described configuration need only be satisfied for two nozzles that are in contact with each other among the plurality of nozzles.

The nozzle assemblies 10, 10A, and 10B are suitably used for suctioning and discharging a washing solution in a BF separation step in an examination using an immunoreaction.

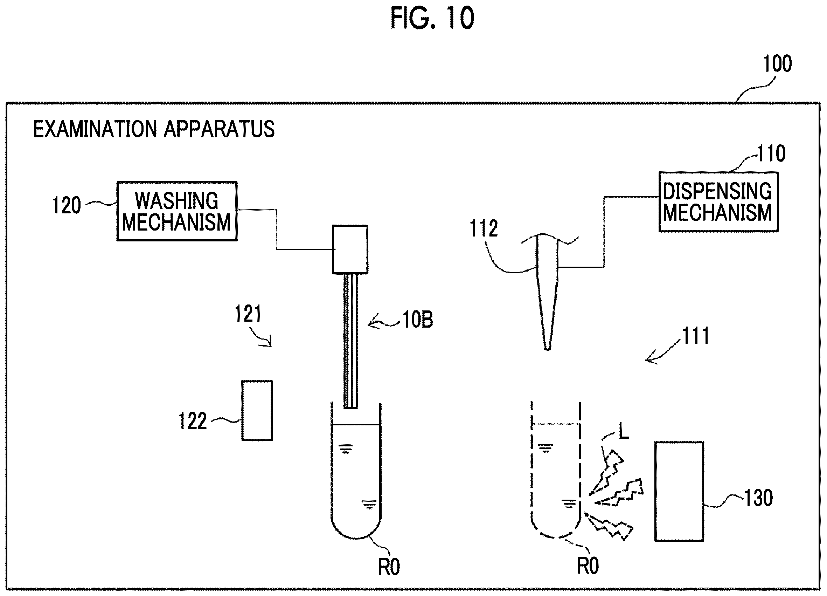

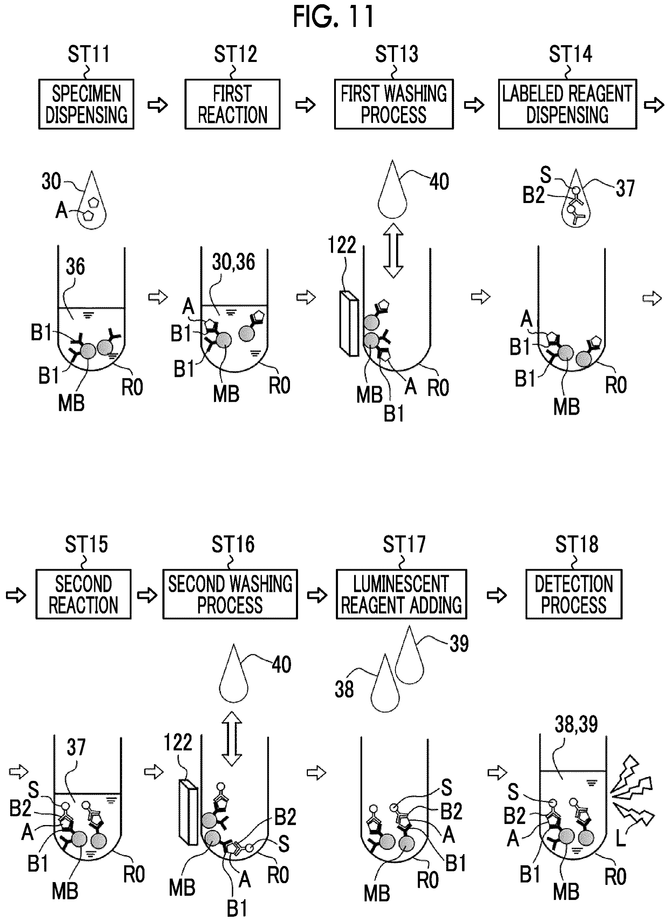

An example of an examination apparatus 100 to which the nozzle assembly 10B is applied will be described with reference to Figs. 10 to 12. Fig. 10 is a schematic diagram showing an overall configuration of an examination apparatus 100 according to one embodiment of the present disclosure. The examination apparatus 100 is an examination apparatus that performs an examination using an immunoreaction, and is an immunoassay apparatus that detects a detection target substance A in a specimen 30 (see Fig. 11) collected from a living body by using an antigen-antibody reaction with magnetic particles as a solid phase. The examination apparatus 100 optically detects the detection target substance A (see Fig. 11) in the specimen 30 using a reaction container R0 and outputs an examination result.

The specimen 30 is, for example, body fluid such as blood collected from a living body. In a case in which the specimen 30 is blood, the specimen 30 may be any of whole blood, blood plasma, serum, and the like. In addition, a centrifuge may be provided in the examination apparatus 100 to extract blood plasma or serum from whole blood. In addition, the detection target substance A that may be contained in the specimen 30 is an antigen, an antibody, a protein, a low-molecular-weight compound, or the like. The specimen 30 is not limited to blood, and need only be a substance collected from a living body, such as urine or body fluid.

The examination apparatus 100 of this example performs an examination based on a chemiluminescent enzyme immunoassay method, for example. As shown in Fig. 10, the examination apparatus 100 comprises, for example, a reaction processing section 111 comprising a dispensing mechanism 110, a washing processing section 121 comprising a washing mechanism 120, and a photodetector 130.

The dispensing mechanism 110 comprises a nozzle 112 for dispensing a reagent. In the reaction processing section 111, the dispensing mechanism 110 dispenses various reagents into the reaction container R0. In the reaction processing section 111, the reagent is dispensed into the reaction container R0, and an immunoreaction occurs in the reaction container R0.

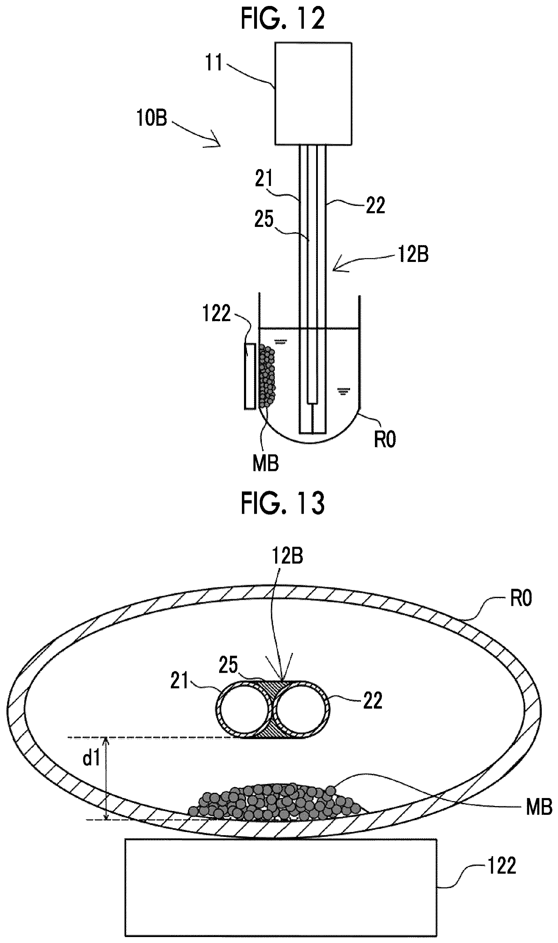

The washing mechanism 120 comprises the nozzle assembly 10B described above and a magnet 122 for magnetic collection. In the washing processing section 121, the washing mechanism 120 suctions and discharges a washing solution 40, which is a BF separation solution, in order to perform BF separation. In the present embodiment, the first nozzle 21 and the second nozzle 22 provided in the nozzle assembly 10B have substantially the same diameter (see Figs. 12 and 13). The first nozzle 21 is used as a suction nozzle, and the second nozzle 22 is used as a washing solution jetting nozzle.

The photodetector 130 is a photomultiplier tube, a photodiode, or the like. The photodetector 130 is disposed to face the reaction container R0 and detects chemiluminescence L caused by a label S bound to the detection target substance A.

The examination apparatus 100 comprises a processor (not shown) that integrally controls the respective units 110 to 130.

A procedure of the chemiluminescent enzyme immunoassay performed in the examination apparatus 100 will be described with reference to Fig. 11. Fig. 11 schematically shows a reaction in the reaction container R0 in a case in which the detection target substance A is contained in the specimen 30.

The reaction container R0 accommodates magnetic particles MB modified with a first binding substance B1 in advance, and a buffer solution 36 is dispensed into the reaction container R0 before the specimen 30 is dispensed. In this state, the specimen 30 is dispensed into the reaction container R0 (step ST11).

In the reaction container R0, the magnetic particles MB, the specimen 30, and the buffer solution 36 are mixed with each other, and as a first reaction, a binding reaction in which the detection target substance A in the specimen 30 and the first binding substance B1 are specifically bound to each other occurs (step ST12). In the first reaction, the detection target substance A in the specimen 30 binds to the first binding substance B1, so that the detection target substance A is captured by the magnetic particles MB via the first binding substance B1.

Next, a first washing process (BF separation) for removing unreacted components other than the detection target substance A captured by the magnetic particles MB is performed (step ST13). In the first washing process, the washing mechanism 120 including the nozzle assembly 10B is used. A reaction liquid after the first reaction is discharged in a state where the magnet 122 is disposed close to the outside of the reaction container R0 and the magnetic particles MB are collected on an inner wall surface of the reaction container R0, and then the washing solution 40 is dispensed into the reaction container R0. In step ST13 in Fig. 11, a bidirectional arrow in an up-down direction shown in an upper portion of the reaction container R0 schematically indicates a state where the washing solution 40 is dispensed into the reaction container R0 and the washing solution 40 is discharged from the reaction container R0. In the first washing process, the dispensing and the discharge of the washing solution 40 are repeated a plurality of times. The discharge of the washing solution 40 from the reaction container R0 is also performed in a state where the magnet 122 is disposed close to the outside of the reaction container R0 and the magnetic particles MB are collected on the inner wall surface of the reaction container R0.

After the first washing process, a labeled reagent 37 is dispensed into the reaction container R0 using the dispensing mechanism 110 (step ST14). The labeled reagent 37 contains a second binding substance B2 to which the label S is attached, which is a binding substance for specifically binding to the detection target substance A.

In the reaction container R0, as a second reaction, a binding reaction occurs in which the detection target substance A captured by the magnetic particles MB and the second binding substance B2 are specifically bound to each other (step ST15). As a result, the label S is attached to the detection target substance A via the second binding substance B2.

Next, a second washing process (BF separation) for removing unreacted components other than the second binding substance B2 bound to the detection target substance A captured by the magnetic particles MB in the labeled reagent 37 is performed (step ST16). The second washing process (step ST16) is performed using the washing mechanism 120 in the same manner as the first washing process (step ST13).

Thereafter, a first luminescent reagent 38 and a second luminescent reagent 39 are added to the reaction container R0 using the dispensing mechanism 110 (step ST17). The label S is an enzyme, and causes the chemiluminescence L in the presence of the first luminescent reagent 38 and the second luminescent reagent 39 containing a luminescent substrate. Then, the chemiluminescence L is detected by the photodetector 130 (step ST18), thereby detecting the detection target substance A.

The above-mentioned chemiluminescent immunoassay method is performed in the examination apparatus 100. That is, in the examination apparatus 100, a pre-process of dispensing various reagents to the respective specimens 30 is performed (steps ST11 to ST17), and a detection process of the chemiluminescence L caused by the label S attached to the detection target substance A is performed (step ST18).

As described above, the nozzle assembly 10B is used in the first washing process (step ST13) and the second washing process (step ST16). As shown in Fig. 12, the nozzle assembly 10B is inserted into the reaction container R0 in a state where the magnet 122 is disposed close to the outside of the reaction container R0 and the magnetic particles MB are collected on the inner wall surface of the reaction container R0. In order to reduce the residual liquid in the reaction container R0 as much as possible in a case of suctioning the liquid, the nozzle assembly 10B is inserted to a position where the tip thereof nearly contacts a bottom surface of the reaction container R0. After the first nozzle 21 suctions the liquid in the reaction container R0, the magnet 122 is separated from the reaction container R0, and the nozzle assembly 10B is raised to a position where the tip thereof is near an upper end of a region where the magnetic particles MB are magnetically collected. Thereafter, the washing solution 40 is discharged from the second nozzle 22, and the magnetic particles MB are dispersed in the washing solution 40 and washed.

In the first washing process and the second washing process, in a case in which a nozzle assembly in which the filling member 25 does not fill the recess portion 14 is used as in a comparative example shown in Fig. 14, the washing solution drawn up into the recess portion 14 may be dried and crystallized while remaining adhered to the recess portion 14. In a case in which the crystals adhering to the recess portion 14 are mixed into the reaction container R0 of the subsequent step or the next measurement object, the measurement accuracy is significantly reduced. In general, after the first washing process and the second washing process, a process of washing the nozzles used in the first washing step and the second washing step in a separate nozzle washing section is performed. In a case in which the liquid is drawn up to the base end side so that it is not subject to washing, the liquid may not be washed and the liquid may be dried and crystallized while remaining adhered.

On the other hand, in the nozzle assembly 10B, the recess portion 14 is filled with the filling member 25 and is flattened, so that it is possible to prevent the liquid from being drawn up due to the capillary action. That is, it is possible to prevent the washing solution 40 from being drawn up, and to suppress the deterioration in measurement accuracy.

The nozzle assembly 10B has a recess portion 14 that does not comprise the filling member 25 at the tip of the nozzle portion 12B (see Fig. 9). Therefore, the liquid may be drawn up into the region having the recess portion 14. However, after the first washing process and the second washing process, a process of washing the used nozzle portion 12B in a separate nozzle washing section is performed, and, in a case in which a region where the recess portion 14 is exposed from the tip of the nozzle portion 12B is within a range of a region to be washed, even in a case in which the liquid adheres to the recess portion 14, the liquid can be removed. As an example, in a case in which the nozzle portion 12B is inserted into the reaction container R0 up to a position of about 15 mm from the tip thereof and comes into contact with the liquid in the nozzle washing section, at least that range is washed by the nozzle washing section. In this case, in a case in which the region that is not filled with the filling member 25 from the tip is a region with the distance D of about 10 mm, the recess portion 14 is washed, and thus crystallization of the liquid or the like does not occur. Therefore, it is possible to avoid a problem in which the crystals adhering to the recess portion 14 are mixed into the reaction container R0 of the subsequent step or the next measurement object, and it is possible to suppress a decrease in measurement accuracy.

Fig. 12 shows a state where the nozzle assembly 10B is inserted into the reaction container R0, and Fig. 13 is a cross-sectional view of the nozzle portion 12B and the reaction container R0 in the state of Fig. 12. As shown in Fig. 13, the reaction container R0 used in the examination apparatus 100 of the present embodiment has an elliptical shape in a plan view. The reaction container R0 has an elliptical shape in a plan view, and the magnetic particles MB are magnetically collected on a wall surface in a minor axis direction, so that the magnetic collection efficiency is high. Since the cross section of the nozzle portion 12B of the nozzle assembly 10B has an oval shape (in this example, a rounded rectangular shape) with respect to such a flat reaction container R0, a sufficient distance can be maintained from the wall surface of the reaction container R0 in a case in which the nozzle portion 12B is inserted into the reaction container R0. As shown in Fig. 13, in a case in which the nozzle portion 12B is inserted into substantially the center of the ellipse of the reaction container R0, there is a distance d1 between the nozzle portion 12B and the reaction container R0. In a state where the magnetic particles MB are magnetically collected on the wall surface of the reaction container R0, the nozzle portion 12B can be inserted into the reaction container R0 without coming into contact with the magnetic particles MB, thereby suppressing erroneous suction of the magnetic particles MB in a case of suctioning the liquid.

For comparison, Fig. 15 shows a case in which a nozzle portion 220 is covered with a holding member 225 that covers the entire outer periphery of the two nozzles 21 and 22 so as to prevent the formation of the recess portion 14 that is generated in a case in which the two nozzles 21 and 22 are brought into contact with each other. As shown in Fig. 15, the nozzle portion 220 of a comparative example has a configuration in which the first nozzle 21 and the second nozzle 22 are inserted into the holding member 225 having a circular cross section. As shown in Fig. 15, in a case in which the nozzle portion 220 is inserted into the substantially center of the reaction container R0 having an elliptical cross section, a distance d2 from the wall surface of the reaction container R0 in the minor axis direction is shorter than the distance d1 in Fig. 13. In a case in which the distance between the nozzle portion 220 and the wall surface is close in this manner, the nozzle portion 220 may come into contact with the magnetic particles MB magnetically collected on the wall surface in a case in which the nozzle portion 220 is inserted, making it easy for the magnetic particles MB to be erroneously suctioned in a case of suctioning the liquid.

As described above, in a case in which the nozzle assembly 10B is used, in the examination apparatus 100 in which the reaction container R0 having an elliptical cross section is used and the magnetic particles MB are used for the BF separation, it is possible to prevent the liquid from being drawn up in the washing process and to suppress the erroneous suction of the magnetic particles MB. As a result, it is possible to effectively suppress the deterioration in measurement accuracy in immunoassay or the like. The same effects can be obtained by using the nozzle assembly 10 or the nozzle assembly 10A instead of the nozzle assembly 10B.

The following appendices are further disclosed with respect to the above embodiment.

### (Appendix 1)

A nozzle assembly in which two nozzles with circular cross sections are integrated in a state of being brought into contact with each other,
in which a recess portion that is generated in a part of an outer periphery of the two nozzles in contact with each other and that has a valley at a contact portion where the nozzles are brought into contact with each other is flattened by being filled with a filling member, and
a part of the outer periphery other than the recess portion, the part including both end parts of the outer periphery in a maximum diameter direction, is not covered by the filling member.

### (Appendix 2)

The nozzle assembly according to Appendix 1,
in which the filling member is provided only in the recess portion.

### (Appendix 3)

The nozzle assembly according to Appendix 2,
in which the nozzle assembly has an oval cross-sectional shape in a state where the recess portion is flattened.

### (Appendix 4)

The nozzle assembly according to any one of Appendices 1 to 3,
in which the two nozzles are made of metal.

### (Appendix 5)

The nozzle assembly according to Appendix 4,
in which the filling member is made of metal.

### (Appendix 6)

The nozzle assembly according to Appendix 5,
in which the metal is silver braze or stainless steel.

### (Appendix 7)

The nozzle assembly according to any one of Appendices 1 to 6,
in which tip positions of the two nozzles are aligned.

### (Appendix 8)

The nozzle assembly according to Appendix 7,
in which the recess portion is filled with the filling member from a position separated from the tip positions by 3 mm or more.

### (Appendix 9)

The nozzle assembly according to any one of Appendices 1 to 8,
in which water-repellent coating is applied to an entire outer periphery including the filling member.

### (Appendix 10)

The nozzle assembly according to Appendix 9,
in which the water-repellent coating is fluorine-based coating.

### (Appendix 11)

The nozzle assembly according to any one of Appendices 1 to 10,
in which the nozzle assembly is used for suctioning and discharging a washing solution in a BF separation step in an examination using an immunoreaction.

### (Appendix 12)

An examination apparatus that performs an examination using an immunoreaction and that performs an examination including a step of, using magnetic particles as a solid phase, binding a detection target substance in a specimen to a binding substance attached to the magnetic particles in a reaction container and then performing BF separation, the examination apparatus comprising:
a reaction processing section that causes an immunoreaction in the reaction container; and
a washing processing section that includes a nozzle assembly for suctioning and discharging a BF separation solution for performing the BF separation,
in which, as the nozzle assembly, the nozzle assembly according to any one of Appendices 1 to 11 is provided.

### (Appendix 13)

The examination apparatus according to Appendix 12,
in which the reaction container has an elliptical shape in a plan view, and
the washing processing section includes a magnet for magnetically collecting the magnetic particles on an inner wall surface of the reaction container in a minor axis direction.

The disclosure of Japanese Patent Application No. 2023-012145 filed on January 30, 2023 is incorporated in the present specification by reference. All documents, patent applications, and technical standards mentioned in the present specification are incorporated herein by reference to the same extent as in a case in which each document, each patent application, and each technical standard are specifically and individually described by being incorporated by reference.

### Explanation of References

10, 10A, 10B: nozzle assembly
11: fixing portion
12, 12A, 12B: nozzle portion
14: recess portion
16: straight line
18: common tangent
21: first nozzle
21a: tip of first nozzle
21b: outer periphery of first nozzle
22: second nozzle
22a: tip of second nozzle
22b: outer periphery of second nozzle
25: filling member
28: water-repellent coat
29: liquid
30: specimen
36: buffer solution
37: labeled reagent
38: first luminescent reagent
39: second luminescent reagent
40: washing solution
100: examination apparatus
110: dispensing mechanism
111: reaction processing section
120: washing mechanism
121: washing processing section
122: magnet
130: photodetector
220: nozzle portion
225: holding member
A: detection target substance
B1: first binding substance
B2: second binding substance
CP: contact portion
D: distance
L: chemiluminescence
MB: magnetic particle
P: tip position
R0: reaction container
S: label

## Claims

1. A nozzle assembly in which two nozzles with circular cross sections are integrated in a state of being brought into contact with each other,
wherein a recess portion that is generated in a part of an outer periphery of the two nozzles in contact with each other and that has a valley at a contact portion where the nozzles are brought into contact with each other is flattened by being filled with a filling member, and
a part of the outer periphery other than the recess portion, the part including both end parts of the outer periphery in a maximum diameter direction, is not covered by the filling member.

2. The nozzle assembly according to claim 1,
wherein the filling member is provided only in the recess portion.

3. The nozzle assembly according to claim 2,
wherein the nozzle assembly has an oval cross-sectional shape in a state where the recess portion is flattened.

4. The nozzle assembly according to claim 1,
wherein the two nozzles are made of metal.

5. The nozzle assembly according to claim 4,
wherein the filling member is made of metal.

6. The nozzle assembly according to claim 5,
wherein the metal is silver braze or stainless steel.

7. The nozzle assembly according to claim 1,
wherein tip positions of the two nozzles are aligned.

8. The nozzle assembly according to claim 7,
wherein the recess portion is filled with the filling member from a position separated from the tip positions by 3 mm or more.

9. The nozzle assembly according to any one of claims 1 to 8,
wherein water-repellent coating is applied to an entire outer periphery including the filling member.

10. The nozzle assembly according to claim 9,
wherein the water-repellent coating is fluorine-based coating.

11. The nozzle assembly according to claim 1,
wherein the nozzle assembly is used for suctioning and discharging a washing solution in a BF separation step in an examination using an immunoreaction.

12. An examination apparatus that performs an examination using an immunoreaction and that performs an examination including a step of, using magnetic particles as a solid phase, binding a detection target substance in a specimen to a binding substance attached to the magnetic particles in a reaction container and then performing BF separation, the examination apparatus comprising:
a reaction processing section that causes an immunoreaction in the reaction container; and
a washing processing section that includes a nozzle assembly for suctioning and discharging a BF separation solution for performing the BF separation,
wherein, as the nozzle assembly, the nozzle assembly according to claim 1 is provided.

13. The examination apparatus according to claim 12,
wherein the reaction container has an elliptical shape in a plan view, and
the washing processing section includes a magnet for magnetically collecting the magnetic particles on an inner wall surface of the reaction container in a minor axis direction.
